# EUROPÄISCHE PATENTSCHRIFT

(11) **EP 3 121 185 B1**
(45) Veröffentlichungstag und Bekanntmachung des Hinweises auf die Patenterteilung: **14.11.2018**
(21) Anmeldenummer: 16180046.1
(22) Anmeldetag: 19.07.2016
(51) Int. Cl.: B01J 31/22, B01J 31/24, C07C 67/38, C07F 17/02, C07F 9/572, C07F 9/655, C07F 9/6553, C07F 15/00, C07F 9/58, C07F 9/6506

(54) **PHOSPHINLIGAND UND DARAUF BASIERENDE PALLADIUM-KATALYSATOREN FÜR DIE ALKOXYCARBONYLIERUNG ETHYLENISCH UNGESÄTTIGTER VERBINDUNGEN**
PHOSPHIN LIGAND AND PALLADIUM CATALYST BASED ON SAME FOR ALKOXYCARBONYLATION OF ETHYLENICALLY UNSATURATED COMPOUNDS
PHOSPHINE ET CATALYSEURS EN PALLADIUM À BASE DE CELLE-CI POUR L'ALKOXY-CARBONYLATION DE LIAISONS INSATURÉES EN ÉTHYLÈNE

(30) Priorität: 23.07.2015 DE 102015213918
(43) Veröffentlichungstag der Anmeldung: 25.01.2017
(73) Patentinhaber: Evonik Degussa GmbH, 45128 Essen (DE)
(72) Erfinder: JENNERJAHN, Reiko, 18190 Sanitz (DE); GÜLAK, Samet, 51373 Leverkusen (DE); FANG, Xiangjie, Shanghai (CN); DONG, Kaiwu, 236800 Bo Zhou (CN); NEUMANN, Helfried, 18055 Rostock (DE); JACKSTELL, Ralf, 27478 Cuxhaven Altenwalde (DE); BELLER, Matthias, 18211 Ostseebad Nienhagen (DE); FRANKE, Robert, 45772 Marl (DE); HESS, Dieter, 45770 Marl (DE); DYBALLA, Katrin Marie, 45657 Recklinghausen (DE); FRIDAG, Dirk, 45721 Haltern am See (DE); GEILEN, Frank, 45721 Haltern am See (DE)

(56) Entgegenhaltungen:
- BIANCHINI C ET AL: "METHOXYCARBONYLATION OF ETHENE BY PALLADIUM(II) COMPLEXES WITH 1,1'-BIS(DIPHENYLPHOSPHINO)FERROCENE (DPPF) AND 1,1'-BIS(DIPHENYLPHOSPHINO)OCTAMETHYLFERRO CENE (DPPOMF)", ORGANOMETALLICS, AMERICAN CHEMICAL SOCIETY, US, Bd. 22, Nr. 12, 9. Juni 2003 (2003-06-09), Seiten 2409-2421, XP001164644, ISSN: 0276-7333, DOI: 10.1021/OM021049B
- IAN R. BUTLER ET AL: ") catalyst precursors. X-Ray structure of [(L-L)HRh( -H) 3 RhH(L-L)]ClO 4 [L-L =rac-Fe([eta] 5 -C 5 H 4 PPhBu t ) 2 ]", JOURNAL OF THE CHEMICAL SOCIETY, CHEMICAL COMMUNICATIONS., no. 11, 1 January 1984 (1984-01-01), pages 719-721, XP55407323, GB ISSN: 0022-4936, DOI: 10.1039/C39840000719

## Beschreibung

Die vorliegende Erfindung betrifft einen verbesserten Phosphinliganden und dessen Verwendung in der Alkoxycarbonylierung.

Die Alkoxycarbonylierung ethylenisch ungesättigter Verbindungen ist ein Prozess mit steigender Bedeutung. Unter einer Alkoxycarbonylierung versteht man die Umsetzung von ethylenisch ungesättigten Verbindungen, wie Olefinen, mit Kohlenmonoxid und Alkoholen in Gegenwart eines Metalls bzw. eines Metallkomplexes und eines Liganden zu den entsprechenden Estern:

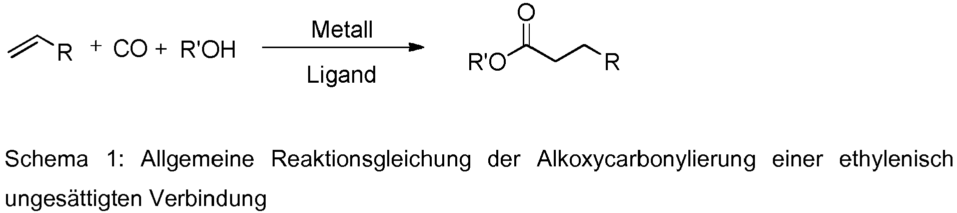

Unter den Alkoxycarbonylierungsreaktionen ist die Ethen-Methoxycarbonylierung zu 3-Methylpropionat von Bedeutung als Zwischenstufe für die Herstellung von Methylmethacrylat (S. G. Khokarale, E. J. García-Suárez, J. Xiong, U. V. Mentzel, R. Fehrmann, A. Riisager, Catalysis Communications 2014, 44, 73-75). Die Ethen-Methoxycarbonylierung wird in Methanol als Lösemittel bei milden Bedingungen mit einem durch Phosphinliganden modifizierten Palladiumkatalysator durchgeführt.

Ein sehr gutes katalytischen System wurde von Lucite - jetzt Mitsubishi Rayon - entwickelt und verwendet einen Liganden auf Basis von 1,2-Bis(di-tert-butylphosphinomethyl)benzol (DTBPMB) (W. Clegg, G. R. Eastham, M. R. J. Elsegood, R. P. Tooze, X. L. Wang, K. Whiston, Chem. Commun 1999, 1877-1878).

Anwendungen der Methoxycarbonylierung auf längerkettige Substrate sind z.B. in EP 0 662 467 A1 beschrieben. Die Patentschrift beschreibt einen Prozess zur Herstellung von Dimethyladipat aus Methyl-3-Pentenoat. Als Pd-Quelle wird Pd(II)acetat verwendet. Als Beispiele geeigneter bidentater Phosphinliganden werden unter anderem 1,1'-Bis(diphenylphosphino)ferrocen, 1-(Diphenylphosphino)-1'-(diisopropylphosphino)ferrocen und 1,1'-Bis(isopropylphenylphosphino)ferrocen genannt. Die Liganden erzielen jedoch nur unzureichende Ausbeuten bei der Methoxycarbonylierung von Olefinen, insbesondere von langkettigen Olefinen wie 2-Octen und Di-n-Buten.

In BIANCHINI C ET AL, "METHOXYCARBONYLATION OF ETHENE BY PALLADIUM(II) COMPLEXES WITH 1,1'-BIS(DIPHENYLPHOSPHINO)FERROCENE (DPPF) AND 1,1'-BIS(DIPHENYLPHOSPHINO)OCTAMETHYLFERROCENE (DPPOMF)", ORGANO-METALLICS, AMERICAN CHEMICAL SOCIETY, US, (20030609), Bd. 22, Nr. 12, Seiten 2409 - 2421, wird der Einsatz von DPPF und DPPOMF zur Katalyse der Methoxycarbonylierung von Ethen beschrieben. Hierbei bilden sich sie folgenden Pd-Komplexe: [Pd(H₂O)₂(dppf)](OTs)₂ bzw. [Pd(H₂O)₂(dppomf)](OTs)₂.

Die technische Aufgabe, welche der vorliegenden Erfindung zugrunde lag, ist die Bereitstellung verbesserter Liganden für Alkoxycarbonylierungsreaktionen. Diese Liganden sollen insbesondere bei der Umsetzung von langkettigen Olefinen wie 2-Octen oder Di-n-Buten verbesserte Ausbeuten erzielen. Insbesondere soll bei der Alkoxycarbonylierungsreaktion die Raum-Zeit-Ausbeute gesteigert werden.

Diese Aufgabe wird gelöst durch ein Verfahren gemäß Anspruch 1.

Es hat sich überraschenderweise gezeigt, dass der erfindungsgemäße Phosphinligand, 1,1'-bis-(tert-butylphenylphosphino)-ferrocen, verbesserte Eigenschaften gegenüber den in EP 0 662 467 A1 beschriebenen, strukturell ähnlichen Liganden aufweist. Insbesondere erzielt der erfindungsgemäße Ligand höhere Ausbeuten bei der Alkoxycarbonylierung langkettiger Olefine wie 2-Octen und Di-n-Buten.

Die Erfindung betrifft ein Verfahren umfassend die Verfahrensschritte:
a) Vorlegen einer ethylenisch ungesättigten Verbindung;
b) Zugabe einer Verbindung gemäß Formel (**1**) 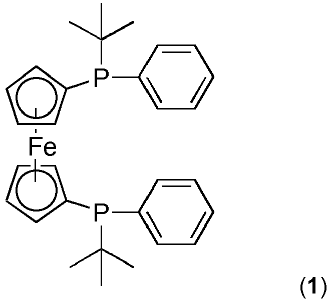 und einer Verbindung, welche Pd umfasst,
   oder Zugabe eines Komplexes umfassend Pd und eine Verbindung gemäß Formel (**1**) 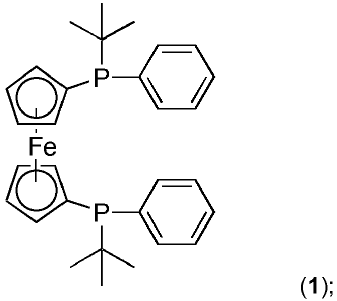
c) Zugabe eines Alkohols;
d) Zuführen von CO;
e) Erwärmen des Reaktionsgemisches, wobei die ethylenisch ungesättigte Verbindung zu einem Ester umgesetzt wird.

Hierbei können die Verfahrensschritte a), b), c) und d) in beliebiger Reihenfolge erfolgen. Üblicherweise erfolgt die Zugabe von CO jedoch, nachdem die Reaktionspartner in den Schritten a) bis c) vorgelegt wurden. Die Schritte d) und e) können gleichzeitig oder nacheinander erfolgen. Darüber hinaus kann CO auch in mehreren Schritten zugeführt werden, so dass beispielsweise zunächst ein Teil des CO zugeführt, dann erwärmt und anschließend ein weiterer Teil CO zugeführt wird.

Die in dem erfindungsgemäßen Verfahren als Edukt eingesetzten ethylenisch ungesättigten Verbindungen enthalten eine oder mehrere Kohlenstoff-Kohlenstoff-Doppelbindungen. Diese Verbindungen werden auch im Folgenden zur Vereinfachung als Olefine bezeichnet. Die Doppelbindungen können terminal oder intern sein.

Bevorzugt sind ethylenisch ungesättigte Verbindungen mit 2 bis 30 Kohlenstoffatomen, bevorzugt 2 bis 22 Kohlenstoffatomen, besonders bevorzugt 2 bis 12 Kohlenstoffatomen.

In einer Ausführungsform umfasst die ethylenisch ungesättigte Verbindung 4 bis 30 Kohlenstoffatome, bevorzugt 6 bis 22 Kohlenstoffatome, weiter bevorzugt 8 bis 22 Kohlenstoffatomen, besonders bevorzugt 8 bis 12 Kohlenstoffatome, am meisten bevorzugt 8 Kohlenstoffatome.

Die ethylenisch ungesättigten Verbindungen können zusätzlich zu der einen oder mehreren Doppelbindungen weitere funktionelle Gruppen enthalten. Vorzugsweise umfasst die ethylenisch ungesättigte Verbindung eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Hydroxyl-, Sulfhydryl- , Amino-, Ether-, Thioether-, Aryl-, Heteroaryl- oder Silylgruppen und/oder Halogensubstituenten. Dabei umfasst die ethylenisch ungesättigte Verbindung vorzugsweise insgesamt 2 bis 30 Kohlenstoffatome, bevorzugt 2 bis 22 Kohlenstoffatome, besonders bevorzugt 2 bis 12 Kohlenstoffatome.

In einer Ausführungsform umfasst die ethylenisch ungesättigte Verbindung keine weiteren funktionellen Gruppen außer Kohlenstoff-Kohlenstoff-Doppelbindungen.

In einer besonders bevorzugten Ausführungsform handelt es sich bei der ethylenisch ungesättigten Verbindung um ein unfunktionalisiertes Alken mit mindestens einer Doppelbindung und 2 bis 30 Kohlenstoffatomen, bevorzugt 6 bis 22 Kohlenstoffatomen, weiter bevorzugt 8 bis 22 Kohlenstoffatomen, besonders bevorzugt 8 bis 12 Kohlenstoffatomen, am meisten bevorzugt 8 Kohlenstoffatomen.

Geeignete ethylenisch ungesättigte Verbindungen sind beispielsweise:
Ethen;
Propen;
C4-Olefine, wie 1-Buten, cis-2-Buten, trans-2-Buten, Gemisch von cis- und trans-2-Buten, Isobuten, 1,3-Butadien; Raffinat I bis III, Crack-C4
C5-Olefine, wie 1-Penten, 2-Penten, 2-Methyl-1-buten, 2-Methyl-2-buten, 2-Methyl-1,3-butadien (Isopren), 1,3-Pentadien;
C6-Olefine, wie Tetramethylethylen, 1,3-Hexadien, 1,3-Cyclohexadien;
C7-Olefine, wie 1-Methylcyclohexen, 2,4-Heptadien, Norbonadien;
C8-Olefine, wie 1-Octen, 2-Octen, Cycloocten, Di-n-Buten, Di-iso-buten, 1,5-Cyclooctadien, 1,7-Octadien;
C9-Olefine, wie Tripropen;
C10-Olefine, wie Dicylcopentadien;
Undecene;
Dodecene;
interne C14-Olefine;
interne C15- bis C18-Olefine;
lineare oder verzweigte, cyclische, acyclische oder teilweise cyclische, interne C15- bis C30-Olefine;
Triisobuten, Tri-n-buten;
Terpene, wie Limonen, Geraniol, Farnesol, Pinen, Myrcen, Carvon, 3-Caren;
mehrfach ungesättigte Verbindungen mit 18 Kohlenstoffatomen, wie Linolsäure oder Linolensäure;
Ester ungesättigter Carbonsäuren, wie Vinylester von Essig- oder Propansäure, Alkylester ungesättigter Carbonsäuren, Methyl- oder Ethylester von Acrylsäure oder Methacrylsäure, Ölsäureester, Ölsäure Methyl- oder Ethylester, Ester der Linol- oder Linolensäure; Vinylverbindungen, wie Vinylacetat, Vinylcyclohexen, Styrol, alpha-Methylstyrol, 2-Isopropenylnaphthalin;
2-Methyl-2-pentenal, Methyl-3-Pentenoat, Methacrylanhydrid.

In einer Variante des Verfahrens ist die ethylenisch ungesättigte Verbindung ausgewählt aus Propen, 1-Buten, cis- und/oder trans-2-Buten, oder Mischungen davon.

In einer Variante des Verfahrens ist die ethylenisch ungesättigte Verbindung ausgewählt aus 1-Penten, cis- und/oder trans-2-Penten, 2-Methyl-1-buten, 2-Methyl-2-buten, 3-Methyl-1-buten, oder Mischungen davon.

In einer bevorzugten Ausführungsform ist die ethylenisch ungesättigte Verbindung ausgewählt aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, n-Octen, 1-Octen, 2-Octen, oder Mischungen davon.

In einer Variante wird ein Gemisch ethylenisch ungesättigter Verbindungen eingesetzt. Als Gemisch wird im Sinne dieser Erfindung eine Zusammensetzung bezeichnet, die wenigsten zwei verschiedene ethylenisch ungesättigte Verbindungen enthält, wobei der Anteil jeder einzelnen ethylenisch ungesättigten Verbindungen vorzugsweise wenigstens 5 Gewichts-% bezogen auf das Gesamtgewicht des Gemisches beträgt.

Vorzugsweise wird ein Gemisch ethylenisch ungesättigter Verbindungen mit jeweils 2 bis 30 Kohlenstoffatomen, bevorzugt 4 bis 22 Kohlenstoffatomen, besonders bevorzugt 6 bis 12 Kohlenstoffatomen, am meisten bevorzugt 8 bis 10 Kohlenstoffatomen eingesetzt. Geeignete Gemische ethylenisch ungesättigter Verbindungen sind die sogenannten Raffinate I bis III. Raffinat I umfasst 40 bis 50 % iso-Buten, 20 bis 30 % 1-Buten, 10 bis 20 % cis- und trans-2-Buten, bis zu 1 % 1,3-Butadien und 10 bis 20 % n-Butan und Isobutan. Das Raffinat II ist ein Teil der beim Naphthacracken entstehenden C₄-Fraktion und besteht im Wesentlichen aus den isomeren *n*-Butenen, Isobutan und *n*-Butan nach Abtrennung von Isobuten aus Raffinat I. Raffinat III ist ein Teil der beim Naphthacracken entstehenden C₄-Fraktion und besteht im Wesentlichen aus den isomeren n-Butenen und n-Butan.

Ein weiteres geeignetes Gemisch ist Di-n-Buten, auch bezeichnet als Dibuten, DNB oder DnB. Di-n-Buten ist ein Isomerengemisch von C8-Olefinen, das aus der Dimerisierung von Gemischen aus 1-Buten, cis-2-Buten und trans-2-Buten entsteht. Technisch werden in der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder teilweise umgesetzt werden. Neben dem dimeren Di-n-Buten, entstehen in der Regel auch höhere Oligomere (Tributen C12, Tetrabuten C16), die nach der Reaktion destillativ abgetrennt werden. Diese können ebenfalls als Edukte eingesetzt werden.

In einer bevorzugten Variante wird ein Gemisch umfassend iso-Buten, 1-Buten, cis- und trans-2-Buten eingesetzt. Vorzugsweise umfasst das Gemisch 1-Buten, cis- und trans-2-Buten.

Die erfindungsgemäße Alkoxycarbonylierung wird durch den erfindungsgemäßen Pd-Komplex katalysiert. Der Pd-Komplex kann dabei in Verfahrensschritt b) entweder als präformierter Komplex umfassend Pd und den erfindungsgemäßen Phosphinliganden zugegeben werden, oder *in situ* aus einer Verbindung, welche Pd umfasst, und dem freien Phosphinliganden gebildet werden. Dabei wird die Verbindung, welche Pd umfasst, auch als Katalysatorvorstufe bezeichnet.

In dem Fall, dass der Katalysator *in situ* gebildet wird, kann der Ligand im Überschuss zugegeben werden, so dass im Reaktionsgemisch auch ungebundener Ligand vorliegt.

Auch im Falle des Komplexes, der gleich zu Beginn zugesetzt wird, kann auch weiterer Ligand zugegeben werden, so dass im Reaktionsgemisch auch ungebundener Ligand vorliegt.

In einer Variante ist die Verbindung, welche Pd umfasst, ausgewählt aus Palladiumdichlorid (PdCl₂), Palladium(II)-acetylacetonat [Pd(acac)₂], Palladium(II)-acetat [Pd(OAc)₂], Dichloro(1,5-cyclooctadiene)palladium(II) [Pd(cod)₂Cl₂], Bis(dibenzylideneaceton)palladium [Pd(dba)₂], Bis(acetonitrile)dichloropalladium(II) [Pd(CH₃CN)₂Cl₂], Palladium(cinnamyl)dichlorid [Pd(cinnamyl)Cl₂]

Vorzugsweise handelt es sich bei der Verbindung, welche Pd umfasst, um PdCl₂, Pd(acac)₂ oder Pd(OAc)₂. Besonders geeignet ist Pd(acac)₂.

Der Alkohol im Verfahrensschritt c) kann verzweigt oder linear sein, cyclisch, alicyclisch, teilweise zyklisch oder aliphatisch und stellt insbesondere ein C₁- bis C₃₀-Alkanol dar. Es können Monoalkohole oder Polyalkohole verwendet werden.

Der Alkohol in Verfahrensschritt c) umfasst vorzugsweise 1 bis 30 Kohlenstoffatome, bevorzugt 1 bis 22 Kohlenstoffatome, besonders bevorzugt 1 bis 12 Kohlenstoffatome. Es kann sich dabei um einen Monoalkohol oder einen Polyalkohol handeln.

Der Alkohol kann zusätzlich zu der einen oder mehreren Hydroxylgruppen weitere funktionelle Gruppen enthalten. Vorzugsweise kann der Alkohol zusätzlich eine oder mehrere funktionelle Gruppen ausgewählt aus Carboxyl-, Thiocarboxyl-, Sulfo-, Sulfinyl-, Carbonsäureanhydrid-, Imid-, Carbonsäureester-, Sulfonsäureester-, Carbamoyl-, Sulfamoyl-, Cyano-, Carbonyl-, Carbonothioyl-, Sulfhydryl-, Amino-, Ether-, Thioether-, Aryl-, Heteroaryl- oder Silylgruppen, und/oder Halogensubstituenten enthalten.

In einer Ausführungsform umfasst der Alkohol keine weiteren funktionellen Gruppen außer Hydroxylgruppen.

Der Alkohol kann ungesättigte und aromatische Gruppen enthalten. Vorzugsweise handelt es sich jedoch um einen aliphatischen Alkohol.

Als aliphatischer Alkohol wird im Rahmen dieser Erfindung ein Alkohol bezeichnet, der keine aromatischen Gruppen umfasst, also beispielsweise ein Alkanol, Alkenol oder Alkinol.

In einer Ausführungsform handelt es sich bei dem Alkohol um ein Alkanol mit einer oder mehreren Hydroxylgruppen und 1 bis 30 Kohlenstoffatomen, bevorzugt 1 bis 22 Kohlenstoffatomen, besonders bevorzugt 1 bis 12 Kohlenstoffatomen, am meisten bevorzugt 1 bis 6 Kohlenstoffatomen.

In einer Variante des Verfahrens ist der Alkohol im Verfahrensschritt c) ausgewählt aus der Gruppe der Monoalkohole.

In einer Variante des Verfahrens ist der Alkohol im Verfahrensschritt c) ausgewählt aus: Methanol, Ethanol, 1-Propanol, Iso-Propanol, Iso-Butanol, tert-Butanol, 1-Butanol, 2-Butanol, 1-Pentanol, 2-Pentanol, 3-Pentanol, 1-Hexanol, Cyclohexanol, Phenol, 2-Ethylhexanol, Isononanol, 2-Propylheptanol.

In einer bevorzugten Variante ist der Alkohol in Verfahrensschritt c) ausgewählt aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

In einer Variante des Verfahrens ist der Alkohol im Verfahrensschritt c) ausgewählt aus der Gruppe der Polyalkohole.

In einer Variante des Verfahrens ist der Alkohol im Verfahrensschritt c) ausgewählt aus: Diolen, Triolen, Tetraolen.

In einer Variante des Verfahrens ist der Alkohol im Verfahrensschritt c) ausgewählt aus: Cyclohexan-1,2-diol, 1,2-Ethandiol, 1,3-Propandiol, Glycerol, 1,2,4-Butantriol,2-Hydroxymethyl-1,3-Propandiol, 1,2,6-Trihydroxyhexan, Pentaerythritol, 1,1,1-Tri(hydroxymethyl)ethan, Brenzkatechin, Resorcin und Hydroxyhydrochinon.

In einer Variante des Verfahrens ist der Alkohol im Verfahrensschritt c) ausgewählt aus: Sucrose, Fructose, Mannose, Sorbose, Galactose und Glucose.

In einer bevorzugten Variante des Verfahrens ist der Alkohol im Verfahrensschritt c) ausgewählt aus Methanol, Ethanol, 1-Propanol, 1-Butanol, 1-Pentanol, 1-Hexanol.

In einer besonders bevorzugten Variante des Verfahrens ist der Alkohol im Verfahrensschritt c) ausgewählt aus: Methanol, Ethanol.

In einer besonders bevorzugten Variante des Verfahrens ist der Alkohol im Verfahrensschritt c) Methanol.

In einer Variante des Verfahrens wird der Alkohol im Verfahrensschritt c) im Überschuss eingesetzt.

In einer Variante des Verfahrens wird der Alkohol im Verfahrensschritt c) gleichzeitig als Lösungsmittel eingesetzt.

In einer Variante des Verfahrens wird ein weiteres Lösungsmittel verwendet, ausgewählt aus: Toluol, Xylol, Tetrahydrofuran (THF) oder Methylenchlorid (CH₂Cl₂).

CO wird in Schritt d) vorzugsweise bei einem CO-Partialdruck zwischen 0,1 und 10 MPa (1 bis 100 bar), bevorzugt zwischen 1 und 8 MPa (10 bis 80 bar), besonders bevorzugt zwischen 2 und 4 MPa (20 bis 40 bar) zugeführt.

Die Reaktionsmischung wird Schritt e) des erfindungsgemäßen Verfahrens vorzugsweise auf eine Temperatur zwischen 10 °C und 180 °C, bevorzugt zwischen 20 und 160 °C, besonders bevorzugt zwischen 40 und 120 °C erwärmt, um die ethylenisch ungesättigte Verbindung zu einem Ester umzusetzen.

Das molare Verhältnis von der in Schritt a) vorgelegten ethylenisch ungesättigten Verbindung zu dem in Schritt c) zugegebenen Alkohol beträgt vorzugsweise zwischen 1:1 bis 1:20, bevorzugt 1:2 bis 1:10, besonders bevorzugt 1:3 bis 1:4.

Das Massenverhältnis von Pd zu der in Schritt a) vorgelegten ethylenisch ungesättigten Verbindung beträgt vorzugsweise zwischen 0,001 und 0,5 Gew.-%, bevorzugt zwischen 0,01 und 0,1 Gew.-%, besonders bevorzugt zwischen 0,01 und 0,05 Gew.-%.

Das molare Verhältnis des erfindungsgemäßen Phosphinliganden zu Pd beträgt vorzugsweise zwischen 0,1:1 und 400:1, bevorzugt zwischen 0,5:1 und 400:1, besonders bevorzugt zwischen 1:1 und 100:1, am meisten bevorzugt zwischen 2:1 und 50:1.

Vorzugsweise wird das Verfahren unter Zusatz einer Säure durchgeführt. In einer Variante umfasst das Verfahren deshalb zusätzlich den Schritt c'): Zugabe einer Säure zum Reaktionsgemisch. Dabei kann es sich vorzugsweise um eine Brønsted- oder eine Lewis-Säure handeln.

Geeignete Brønsted-Säuren haben vorzugsweise eine Säurestärke von pKₛ ≤ 5, bevorzugt eine Säurestärke von pKₛ ≤ 3. Die angegebene Säurestärke pKₛ bezieht sich auf den bei Normalbedingungen (25°C, 1,01325 bar) bestimmten pKₛ-Wert. Bei einer mehrprotonigen Säure bezieht sich die Säurestärke pKₛ im Rahmen dieser Erfindung auf den pKₛ-Wert des ersten Protolyseschrittes.

Vorzugsweise ist die Säure keine Carbonsäure.

Geeignete Brønsted-Säuren sind beispielsweise Perchlorsäure, Schwefelsäure, Phosphorsäure, Methylphosphonsäure und Sulfonsäuren. Vorzugsweise handelt es sich bei der Säure um Schwefelsäure oder eine Sulfonsäure. Geeignete Sulfonsäuren sind beispielsweise Methansulfonsäure, Trifluormethansulfonsäure, tert-Butansulfonsäure, p-Toluolsulfonsäure (PTSA), 2-Hydroxypropan-2-sulfonsäure, 2,4,6-Trimethylbenzolsulfonsäure und Dodecylsulfonsäure. Besonders bevorzugte Säuren sind Schwefelsäure, Methansulfonsäure, Trifluormethansulfonsäure und p-Toluolsulfonsäure.

Als Lewis-Säure kann beispielsweise Aluminiumtriflat eingesetzt werden.

In einer Ausführungsform beträgt die Menge an in Schritt c') zugesetzter Säure 0,3 bis 40 mol-%, bevorzugt 0,4 bis 15 mol-%, besonders bevorzugt 0,5 bis 5 mol-%, am meisten bevorzugt 0,6 bis 3 mol-%, bezogen auf die Stoffmenge der in Schritt a) eingesetzten ethylenisch ungesättigten Verbindung.

### Beispiele

Die nachfolgenden Beispiele veranschaulichen die Erfindung.

### Allgemeine Arbeitsvorschriften

Alle nachfolgenden Präparationen wurden mit Standard-Schlenk-Technik unter Schutzgas durchgeführt. Die Lösungsmittel wurden vor Gebrauch über geeigneten Trocknungsmitteln getrocknet (Purification of Laboratory Chemicals, W. L. F. Armarego (Autor), Christina Chai (Autor), Butterworth Heinemann (Elsevier), 6. Auflage, Oxford 2009).

Phosphortrichlorid (Aldrich) wurde vor dem Einsatz unter Argon destilliert. Alle präparativen Arbeiten erfolgten in ausgeheizten Gefäßen. Die Charakterisierung der Produkte erfolgte mittels NMR-Spektroskopie. Chemische Verschiebungen (δ) werden in ppm angegeben. Die Referenzierung der ³¹P-NMR-Signale erfolgte gemäß: SR_{31P} = SR_{1H} * (BF_{31P} / BF_{1H}) = SR_{1H} * 0,4048. (Robin K. Harris, Edwin D. Becker, Sonia M. Cabral de Menezes, Robin Goodfellow, und Pierre Granger, Pure Appl. Chem., 2001, 73, 1795-1818; Robin K. Harris, Edwin D. Becker, Sonia M. Cabral de Menezes, Pierre Granger, Roy E. Hoffman und Kurt W. Zilm, Pure Appl. Chem., 2008, 80, 59-84).

Die Aufnahme von Kernresonanzspektren erfolgte an Bruker Avance 300 bzw. Bruker Avance 400, die gaschromatografische Analyse an Agilent GC 7890A, die Elementaranalyse an Leco TruSpec CHNS und Varian ICP-OES 715, und die ESI-TOF Massenspektrometrie an Thermo Electron Finnigan MAT 95-XP und Agilent 6890 N/5973 Geräten.

### Herstellung von 1,1'-bis-(tert-butylphenylphosphino)-ferrocen

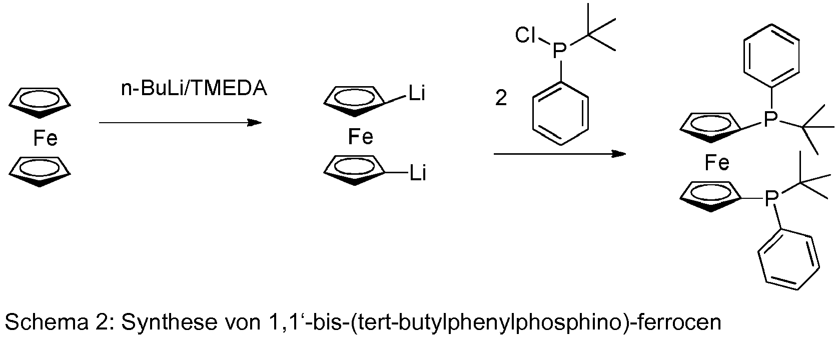

In einem 250 ml Dreihalskolben wurden 3 g Ferrocen unter Argon in 70 ml Heptan gelöst. Es wurden 5,3 ml Tetramethylethylendiamin (TMEDA) und anschließend 22 ml 1,6 N Butyllithium in Hexan hinzugegeben. Die Reaktionsmischung wurde bei Raumtemperatur 24 Stunden stehen gelassen, anschließend wurde das überstehende Lösungsmittel abgenommen. Der Rückstand wurde mit 70 ml Heptan versetzt. In 20 ml Heptan wurden 6,1 ml Chlor-tert-butyl-phenyl-phosphin gelöst und diese Lösung zu der Lösung im Dreihalskolben getropft. Die Reaktionsmischung wurde 1 Stunde lang erhitzt, anschließend auf Raumtemperatur abgekühlt und wässrig aufgearbeitet. Die organische Phase wurde bis ins Trockene eingeengt. Das Produkt wurde aus heißem Methanol kristallisiert und filtriert. Die Ausbeute betrug 4 g.
³¹P NMR (CD₂Cl₂, 121 MHz), s 8.1 ppm
¹H NMR (CD₂Cl₂, 300 MHz), 7,65 -7,55 m (4 H), 7,35- 7,25 m (6H), 4,2-4,15 m(1 H), 4,2-4,15 m (1H), 4,15-4,0 m (1 H), 4,0-3,95 m (2 H), 3,9-3,85 m (3 H), 0. 8 d J = 12, 4 Hz, (18 H)
¹³C (CD₂Cl₂, 75 MHz), 137,7 d, J = 14 Hz, 137,4 d , J = 12,4 Hz, 136,1 d, J = 1,9 Hz, 135,8 d, J = 1,5 Hz, 129,3 d, J = 4,2 Hz, 127,9 d, J = 9 Hz, 77,6 d, J = 33,9 Hz, 77,0 d = 31,8 Hz, 75,1 d, J = 16,8 Hz, 72,9 s, 72,8 s, 72,6 s, 72-71,8 m, 31-30,8 m, 28,2 d, J = 14,8 Hz

### Alternativer Syntheseweg zu Verbindung 1

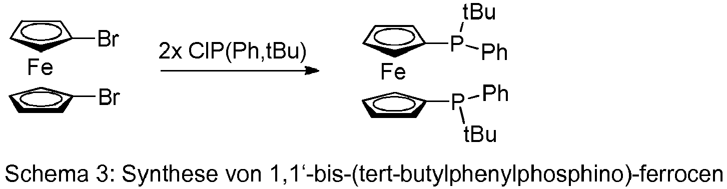

1,1'-Dibromferrocen (5.22 g, 15.2 mmol) wurde in 50 mL THF vorgelegt. Bei -60 °C wurde dann innerhalb von ca. 15 min eine Butyllithium-Lösung (9.5 mL, 15.2 mmol, 1.6 M in Hexan) zugetropft. Die Reaktionslösung wurde anschließend für ca. 15 min weiter gerührt und dabei leicht erwärmt (-30 °C). Dann wurde wieder auf -60 °C abgekühlt, innerhalb weiterer ca. 15 min eine Chlor-tert-butyl-phenyl-phosphin-Lösung (3.05 g, 15.2 mmol, in 10 mL THF) zugegeben und anschließend auf Raumtemperatur erwärmt. Dies wurde einmal wiederholt. Nach der Reaktion wurde das Lösungsmittel entfernt und durch Diethylether (50 mL) ersetzt. Dann wurde zweimal mit 25 mL H₂O gewaschen und über MgSO₄ getrocknet. Durch Einengen fällt ein rostfarbener Niederschlag aus, welcher mit kaltem Diethylether gewaschen wurde. Es konnte 7.28 g (71%) Produkt erhalten werden. Analytik siehe vorhergehender Versuch.

### Herstellung von 1-(diphenylphosphino)-1'-(di-isopropylphosphino)-ferrocen (Vergleichsverbindung)

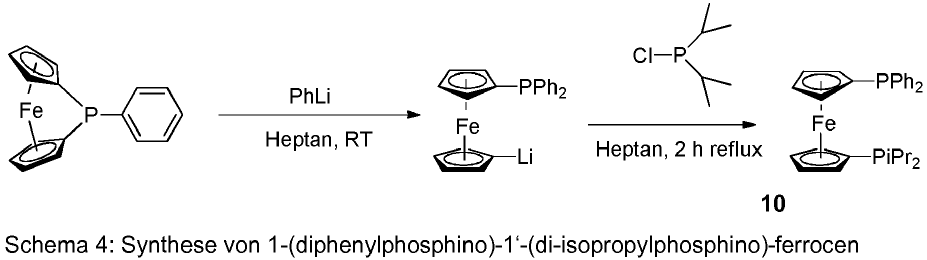

In einen 50 mL Rundkolben mit Magnetrührkern und Stickstoffansatz werden 1,13 mmol (565 µl) Phenyllithium (PhLi) vorgelegt und eine Lösung aus 1,03 mmol (300mg) cyclischen Phosphan in 20 ml Heptan langsam via Spritzenpumpe zugetropft. Das Li-Salz wird zweimal mit Heptan gewaschen und mit 6 ml Heptan versetzt. Zur Suspension wird eine Heptanlösung von 0,8 eq (0,824 mmol, 131 µl) ClP*i*Pr₂ in 7 ml Heptan bei Raumtemperatur zugetropft. Die rotbraune Suspension verfärbt sich kaum. Nach 20 min Rühren wird die Suspension unter Rückfluss 1,5 Stunden erwärmt. Der Feststoff hellt sich etwas auf. Lösungsmittel wird vollständig entfernt und der braun-rote Rückstand wird in H₂O und Ether aufgenommen. Die org. Phase wird zweimal mit H₂O gewaschen und über Na₂SO₄ getrocknet. Von der Etherphase wird ein ³¹P-Spektrum aufgenommen. Das Spektrum zeigt 2 Singuletts. Das Chlorphosphin ist vollständig verbraucht worden. Die Etherphase wird zur Trockene gebracht und man erhält 300 mg (Ausbeute: 61%) eines braungelben Öls, das sich in MeOH auf dem Wasserbad bei 65 °C gelöst. Die Lösung wird über Nacht in den Tiefkühler (-78 °C) gestellt. Es fallen 76 mg eines braungelben Öles aus, das NMR-spektroskopisch untersucht wird.
¹H NMR (300 MHz, CDCl₃) δ 7.46-7.23 (m, 10H, Ph), 4.36 (m, 2H, Cp), 4.21 (m, 2H, Cp), 34.24 (m, 4H, Cp), 1.88 (m, 2H, iPr), 1.15-0.96 (m, 12H, iPr).
¹³C NMR (75 MHz, CDCl₃) δ 139.9 (J = 9.8 Hz, Ph), 133.4 (J = 19.2 Hz, Ph), 128.4, 128.1, 128.0 (Ph), 77.1, 76.8, 76.2, 76.1 (Cp), 73.5 (J = 14.5 Hz, Cp), 72.8 (J = 2.9 Hz, Cp), 71.9 (J = 10.5 Hz, Cp),72.1 (Cp), 23.3 (J = 11.0 Hz, iPr), 20.1, 20.0, 19.9, 19.8 (iPr).
³¹P NMR (121 MHz, C₆D₆) δ = 0.88 und -16.62

### Herstellung von 1-(diphenylphosphino)-1'-(dicyclohexylphosphino)-ferrocen (Vergleichsverbindung)

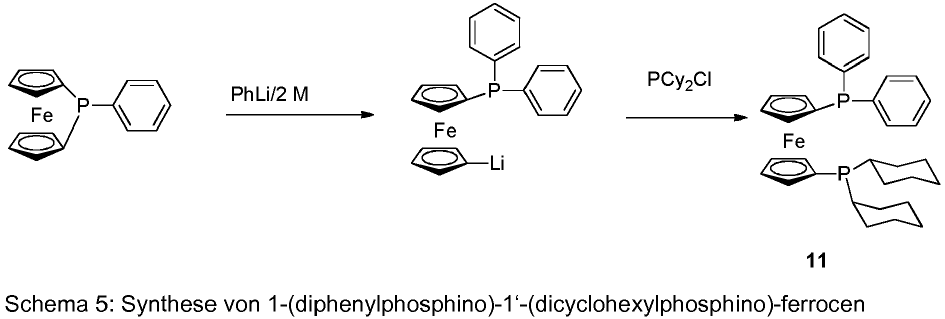

### Chemikalienansatz: 0,6 ml 2 M /THF Phenyllithium, 0,25 ml Chlordicyclohexylphosphin, 50 ml Heptan, 20 ml THF, 30 ml Diethylether, 20 ml Methanol

Der Ausgangsstoff wurde in 30 ml Heptan in einem 100 ml-Schlenkkolben gelöst. In eine 100ml Dreihalskolben wurde das Phenyllithium vorgelegt. Die Lösung wird nun langsam zum Phenyllithium zugetropft und danach 1 Stunde stehen gelassen. Die Reaktionslösung wird dann auf ungefähr 15 ml eingeengt. Dann wird es 30 min stehen gelassen. Die überstehende Flüssigkeit wird abgenommen. Zu dem Rückstand gibt man 20 ml Heptan. Das PCy₂Cl wird in 20 ml THF gelöst und innerhalb einer halben Stunde zugegeben. Die Reaktionslösung wird nun 1h über Rückfluss gekocht. Die Reaktionslösung wird nun auf Raumtemperatur abkühlt und ein Lösungsmittelwechsel zu Diethylether vorgenommen. Dann wird wässerig aufgearbeitet. Der Et₂O wird abgezogen. Der Rückstand wird im heißen Methanol gelöst. Die noch heiße Lösung filtrieren. Es fällt im kalten Zustand ein gelber Niederschlag aus. Die Ausbeute beträgt 300 mg.
³¹P(CDCl₃, 121 MHz), -7,2 s, -16,8 s
¹³C(75 MHz, CDCl₃), 133,4 (d, J = 19,3 Hz), 128,5 s, 128,2 (d, J = 6,8 Hz), 73,6 (d, J = 14,4 Hz), 72,9 (d, J = 4,9 Hz), 72,3 (d, J = 10,5 Hz), 71,2 bs, 69,1 s, 33,5 (d, J = 11,5 Hz), 30,4 (d, J = 4,9 Hz), 30,2 (d, J = 10,2 Hz), 27,4 (dd), 26,4 s
¹H (300 MHz, CDCl₃), 7,3-7,2 m (10 H), 4,2 (t, J = 1,7 Hz, 2H), 4,13 t, J = 1,7 Hz, 2H), 3,98 (quint, J = 1,8 Hz, 4H), 1,8-1,5 (m, 11H), 1,8-1,2 m, 11 H)

### Herstellung von 1-(diphenylphosphino)-1'-(diadamantylphosphino)-ferrocen (Vergleichsverbindung)

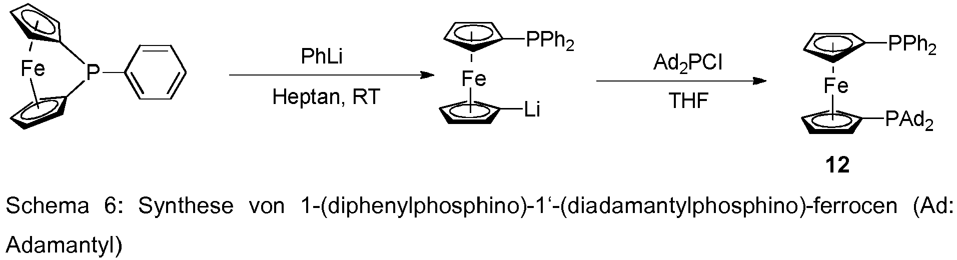

In einem 100 ml Rundkolben mit Magnetrührkern und Stickstoffansatz werden 565 µl (1,13 mmol, 1,1 eq) PhLi (2M) vorgelegt. Innerhalb 2 Stunden wird eine Lösung aus 300 mg (1,03 mmol) verbrücktes Phosphin und 20 ml Heptan mit einer Spritzenpumpe (0,1 Faktor 10, 10 ml Spritze) zugetropft. Nach Zugabe von 1/3 Lösung fällt ein oranger Feststoff aus. Nach Zutropfen wird noch eine Stunde weitergerührt. Die dunkle Lösung wird dekantiert und zweimal mit je 6 ml Heptan gewaschen. Zum orangen Feststoff werden 6 ml Heptan zugegeben und bei 0 °C wird zur Suspension das Chlorphosphin (1,1 eq.), gelöst in 7 ml THF, zugetropft. Über Nacht lässt man unter Rühren die Lösung auf Raumtemperatur aufwärmen. Die Suspension hat sich zu einer orangen roten klaren Lösung verändert. Nach Zugabe von 200 µl Wasser wird die Lösung zur Trockne gebracht und der Rückstand in Ether und Wasser aufgenommen. Die wässrige Phase ist stark alkalisch (pH-Papier) Nach Waschen der roten organischen Phase wird zweimal mit Wasser gewaschen und über Na₂SO₄ getrocknet. Nach Abziehen des Ethers, bleibt ein orangener Feststoff zurück.

Zur Vervollständigung der Reaktion wurde 2 Stunden unter Rückfluss erhitzt. Die Menge an Ad₂PCl wurde von 1.1 eq auf 1.0 eq gesenkt. Gemäß ³¹P-NMR hat sich trotz Rückfluss nicht mehr umgesetzt. Nach Integration der Phosphorsignale erhielt man 50mol% gewünschtes Produkt 28 mol% Ad₂PCl und vermutlich 13 mol% Monoprodukt. Aus der Lösung sind rote Kristalle ausgefallen (Ausbeute von roten Kristallen 160 mg). Es handelt sich um das gewünschte Zielprodukt.
³¹P NMR δ = 26.0, -17.0;
ESI: C₄₂H₄₉FeP₂: ber. 671.26542, gefunden 671.2664
¹H NMR (300 MHz, C₆D₆) δ 7.31-7.13 (m, 10H, Ph), 4.28 (m, 2H, Cp), 4.19 (m, 2H, Cp), 4.01 (m, 2H, Cp), 3.99 (m, 2H, Cp), 1.94-1.61 (m, 18H, Ad), 1.54 (m, 12H, Ad).
³¹P NMR (121 MHz, C₆D₆) δ = 26.04 und -17.00.

### Herstellung von 1-(diphenylphosphino)-1'-(tert-butylphenylphosphino)-ferrocen (Vergleichsverbindung)

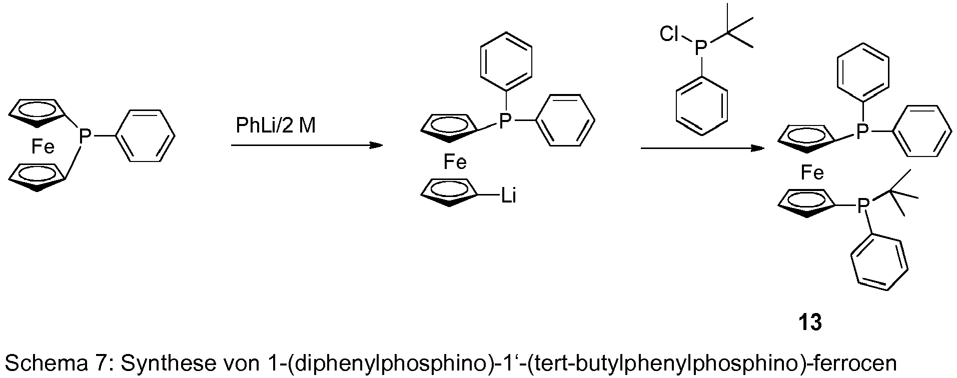

### Chemikalienansatz:

### 0,53 ml 2 M /THF Phenyllithium, 0,19 ml tert-Butylchlor(phenyl)phosphin, 50 ml Heptan, 20 ml THF, 30 ml Diethylether, 20 ml Methanol

Der Ausgangsstoff wurde in 30 ml Heptan in einen 100 ml Schlenkkolben gelöst. In einem 100 ml Dreihalskolben wurde das Phenyllithium vorgelegt. Die Lösung wurde bei -70 °C langsam zum Phenyllithium zugetropft und danach 1 Stunde stehen gelassen. Die Reaktionslösung wird dann auf ungefähr 15 ml eingeengt. Dann wird es 30 min stehen gelassen. Die überstehende Flüssigkeit wird abgenommen. Auf den Rückstand kommen 20 ml Heptan. Das tert-Butylchlor(phenyl)phosphin wird in 20 ml THF gelöst und innerhalb einer halben Stunde zugegeben. Die Reaktionslösung wird nun 1h über Rückfluss gekocht. Die Reaktionslösung wird nun auf Raumtemperatur abkühlt und ein Lösungsmittelwechsel zu Diethylether vorgenommen. Dann wird wässerig aufgearbeitet. Der Et₂O wird abgezogen. Der Rückstand wird im heißen Methanol gelöst. Die noch heiße Lösung filtrieren. Es fällt im kalten Zustand ein gelber Niederschlag aus.
³¹P (CDCl₃ (121 MHz)) 8,1 s, -16,8 s

### Herstellung von 1,1'-bis-(iso-propylphenylphosphino)-ferrocen (Vergleichsverbindung)

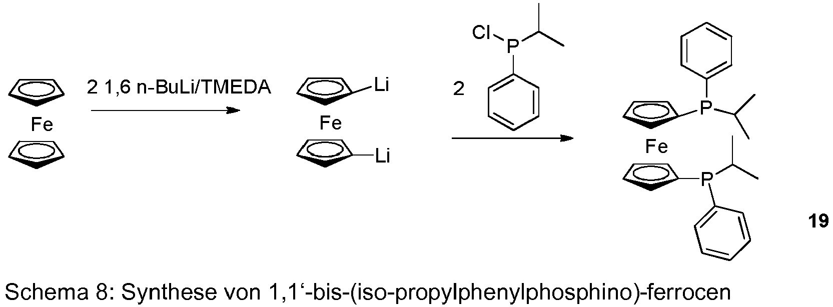

0,93 g Ferrocen werden in 50 ml absolutem Heptan in einem 100 ml Dreihalskolben, versehen mit einem Thermometer, Magnetrührer und Rückflusskühler, gelöst. Es werden 1,3 g TMEDA (1,6 ml) und 7,5 ml 1,6 n-BuLi/Hexan bei Raumtemperatur mittels Spritzen zugesetzt. Die Lösung wird 5 Stunden stehen gelassen. Es fallen große orangebraune Kristalle des dilithiierten Ferrocens aus. Die überstehende Lösung wird mittels einer Spritze entfernt. Und es werden 20 ml absolutes Heptan zugesetzt. Anschließend wird das Chlorphosphin gelöst in 10 ml Heptan zugetropft. Es wird ein Stunde unter Rückfluss erwärmt. Nach dem Abkühlen wird Die organische Phase dreimal mit jeweils 10 ml entgastem Wasser gewaschen. Es wird ins Trockene eingeengt und es werden 10 ml Diethylether zugesetzt. Diese Lösung wird mit Diethylether als Lösungsmittel durch 10 cm Kieselgel 60 unter Argon filtriert, eingeengt ins Trockene und aus wenig heißem Methanol kristallisiert das Zielprodukt in ca. 50 %-iger nicht optimierter Ausbeute.
³¹P (121 MHz, CDCl₃), - 7,8 s, - 8,15 s,
¹³C (75 MHz, CDCl₃);137,77, (d, J = 12 Hz), 137,4 (d, J = 11,3 Hz), 134,2 (d, J = 20,3 Hz), 129,1 s, 128,1 (d, J = 7,5 Hz), 77,4 (d, J = 11,3 Hz), 75,0 (d, J = 26,2 Hz), 74,0 (d, J = 22,3 Hz), 72,1 bs, 71,9-71,5 m, 71,1 s, 69,0 s, 27,6 (d, J = 10 Hz), 27,55 8d, J = 10 Hz), 20,3-19,9 m
¹H (300 MHz, CDCl₃): 7,52-7,44 (m, 4H), 7,33-7,23 (m, 6H), 4,23 (sept, J = 1,2 Hz, 1H), 4,1-4,0 (m, 4 H), 3,93-3,9 (m, 1H), 3,87-3,84 (m, 1H), 3,58-3,54 (m, 1H),
2,1-1,9 (m, 2 H), 0,99 (d, J = 7 Hz, 3H), 0,94 (d, J = 7 Hz, 3 H), 0,83-0.7 (m, 6H)

### Herstellung von 1,1'-bis-(di-(1,4-di-trifluormethyl-phenyl)-phosphino)-ferrocen (Vergleichsverbindung)

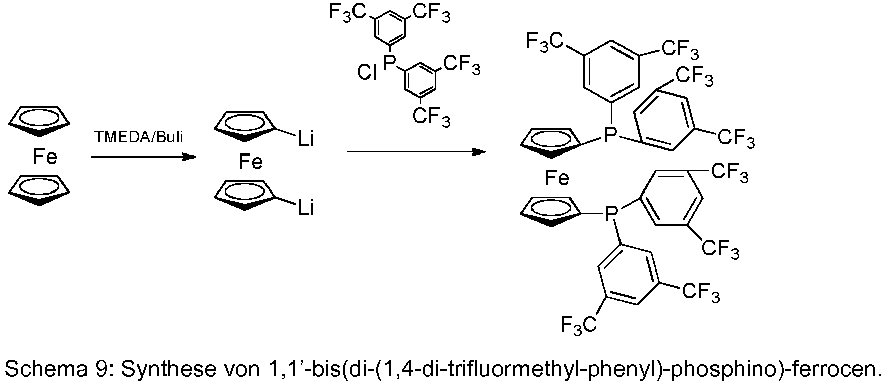

### Chemikalienansatz: 0,3 g Ferrocen, 0,61 ml TMEDA, 2,45 ml Butyllithium, 1,75 ml PClR₂

Das Ferrocen wird im 100 ml Dreihalskolben in 15 ml Heptan gelöst. Dann gibt man TMEDA zu. Das Butyllithium kommt ebenfalls nach der TMEDA-Zugabe dazu. Der 100 ml Kolben mit der Reaktionsmischung bleibt bei Raumtemperatur 24 h stehen. Nach den 24 h nimmt man das überstehende Lösungsmittel ab. Auf den Rückstand kommen dann 20 ml Heptan. Zunächst löst man PClR₂ in 10 ml Heptan. Dieses wird dann in den Kolben getropft. Die Reaktion wird dann 30 min bei 70 °C erhitzt. Danach auf Raumtemperatur abgekühlt und wäßrig aufgearbeitet. Die organische Phase wird bis zu Trockne eingeengt und dann aus heißem Methanol kristallisiert. Ausbeute: 850mg

Analytik:
³¹P (CD₂Cl₂; 121 MHZ), -14,7 s,
HRMS berechnet für 1129,00242, gefunden: 1129.00236
MS EI 70eV) M/z (%), 1098 (M+, 17) 930 (63), 670(81), 473(100), 319(9,88) 261(16), 195(71), 97 (13), 69(23)

### Weitere Vergleichsverbindungen

Die nachfolgend verwendeten Vergleichsverbindungen 1-(di-tert-butylphosphino)-1'-(diphenylphosphino)-ferrocen und 1,2-bis-(di-tert-butyl-phosphino-methyl)-benzol sind kommerziell verfügbar.

### Herstellung von [Pd((Cp₂Fe)1,1'-(PPh(t-butyl))₂)(η²-Styrol)] (Phenyl-η²-ethen) (Komplex K1)

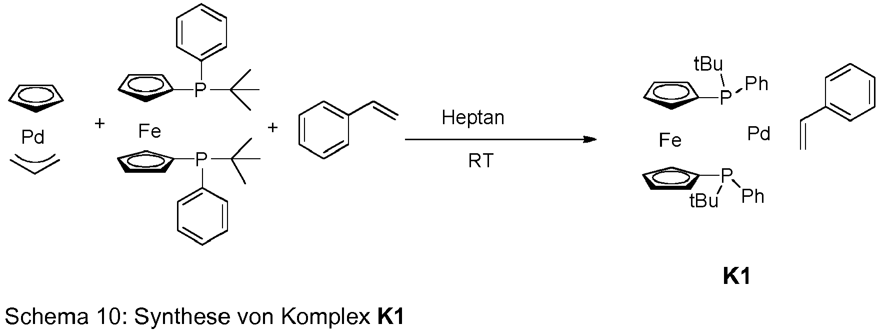

109,6 mg (0,517 mmol) (η³-Allyl)-(η⁵-Cyclopentadienyl)palladium wird in ca. 10 ml Heptan gelöst und über Celite filtriert. In die klare tiefrote Lösung werden 250,8 mg (0,486 mmol) Ferrocenligand - gelöst in 10 ml Heptan - zusammen mit 55,8 µl (0,486 mmol) Styrol bei Raumtemperatur zu getropft. Die dunkelrote Lösung hellt sich nach einigen Minuten Rühren auf. Nach einer Stunde wird eine NMR-Probe abgeschmolzen und ein ³¹P NMR aufgenommen. Der Ligand hat sich vollständig umgesetzt. Die Lösung lässt man vier Stunden weiterrühren und bewahrt sie dann bei -28 °C auf. Es bilden sich keine Kristalle. Die Reaktionslösung wird dann im Hochvakuum stark eingeengt und ein gelber Feststoff fällt aus. Zur Vervollständigung der Reaktion wird die Suspension über Nacht bei -28 °C in den Tiefkühler gestellt. Die überstehende Lösung wird dekantiert und der gelbe Feststoff wird drei Mal mit Heptan gewaschen und an der Ölpumpe getrocknet. Ausbeute: 40-60 %. Das Phosphorspektrum zeigt zwei große Signale, die jeweils leicht zu Dupletts aufgespalten sind, und zwei breitere Signale.
¹H NMR (300 MHz, C₆D₆) δ 8,53-6,71 (m, 10H, Ph), 5,03-3,27 (m, 8H, Cp), 1,47-1,04 (m, 18H, tBu).
³¹P NMR (121 MHz, C₆D₆) δ = 44,91 (d, J = 20,3 Hz), 41,27 (d, J = 20,3 Hz), 44,01 (br), 39,89 (br).

Die Phosphorsignale weisen darauf hin, dass aufgrund diastereomerer Liganden auch diastereomere Komplexe entstanden sind.

### Herstellung von [Pd((Cp₂Fe)1,1'-(PPh(t-butyl))₂)(η²-Naphthochinon)] (Komplex K2)

Für die Komplexsynthese wurde Naphthochinon eingesetzt, um einen gut zu kristallisierenden Komplex zu erhalten.

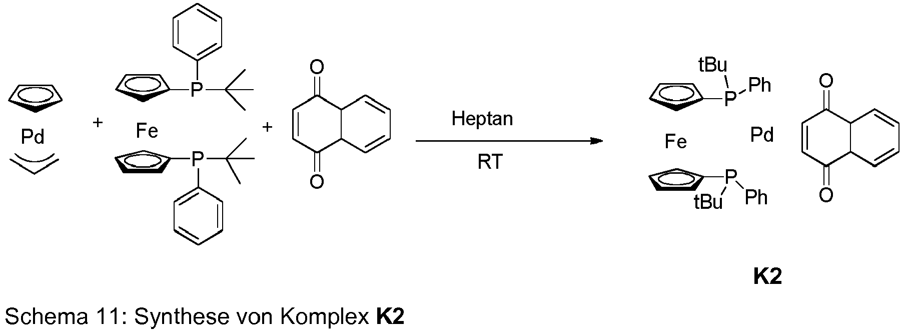

Wichtig bei dieser Reaktion ist, dass man das Naphthochinon vor der Reaktion sublimiert (100 °C und 1.10⁻³ mbar). Sublimiertes Naphthochinon ist ein gelber kristalliner Feststoff.
109,6 mg (0,516 mmol) Palladiumprecursor (siehe Schema 11) werden in 10 ml Heptan gelöst und über Celite in ein 25 ml-Rundkolben filtriert. Zu der tiefroten klaren Lösung werden 250,8 mg (0,487 mmol) Ferrocenligand und 76,86 mg (0,485 mmol, 1,1 eq) frisch sublimiertes Naphtochinon (gelöst in 15 ml Heptan) bei Raumtemperatur zugetropft. Die Lösung mit Ligand und Naphtochinon ist klar und orange gefärbt. Für die vollständige Auflösung des Naphthochinons muss die Lösung auf 60 °C mit dem Wasserbad erwärmt werden (eventuell mit dem Ultraschallbad die vollständige Lösung erzielen). Die Reaktionslösung hellt sich auf und es fällt ein rotbrauner Feststoff aus. Man lässt über Nacht rühren. Nach dem Absetzen dekantiert man die Lösung und wäscht den rotbraunen Feststoff zwei Mal mit Heptan. Nach dem Trocknen im Vakuum erhält man einen rotbraunen Feststoff (50-70%).

Es handelt sich um eine Isomerenmischung, die im ³¹P-NMR 6 verschiedene Phosphorsignale zeigt und auf 3 diastereomere Komplexe hinweist.
¹H NMR (300 MHz, C₆D₆) δ 8,40-8,27 (m, 1,5H, CH arom), 8,17 (m, 0,5H, CH arom), 8,09-87,99 (m, 1H, CH arom), 7,69-7,59 (m, 1H, CH arom), 7,49 (m, 1H, CH arom), 7,30-7,12 (m, 7H, CH arom), 7,08 (m, 1H, CH arom), 6,94-6,66 (m, 3H, CH arom), 5,21-4,98 (m, 2H, CH vinyl), 4,34 (m, 0,5H, Cp), 4,10 (m, 1H, Cp), 4,02 (m, 1H, Cp), 3,95 (m, 0,5H, Cp), 3,90-3,81 (m, 1H, Cp), 3,79 (m, 0,5H, Cp), 3,75 (m, 1H, Cp), 3,66 (m, 0,5H, Cp), 3,60 (m, 1H, Cp), 3,48 (m, 1H, Cp), 1,26-1,06 (m, 18H, tBu).
³¹P NMR (121 MHz, C₆D₆) δ = 50,89 (d, Hz, J = 25,8 Hz), 47,37 (d, Hz, J = 25,8 Hz), 49,58 (s), 46,73 (s).

Elementaranalyse berechnet für C₄₀H₄₂FeO₂P₂Pd: C, 61,67; H, 5,43; P,7,95. Gefunden: C, 61,36; H, 5,56; P, 7,65.

### Herstellung von [Pd((Cp₂Fe)1,1'-(PPh(t-butyl))₂)(η²-N-Methylmaleinimid)] (Komplex K3)

Ebenso kann der Palladiumkomplex auch mit N-Methylmaleinimid hergestellt werden:

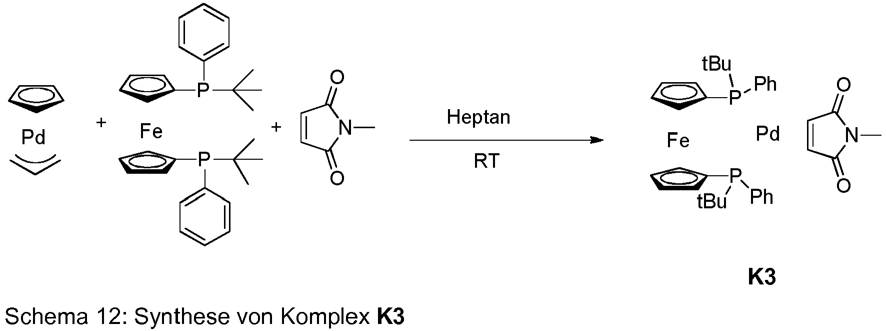

Wichtig bei dieser Reaktion ist, dass man das Maleinimid vorher sublimiert (bei 100 °C und 1·10⁻³ mbar). Sublimiertes N-Methylmaleinimid ist ein weißer kristalliner Feststoff. Analog zur Synthese mit Naphthochinon (siehe Versuch 50) erhält man 287 mg (80%) Produkt als einen gelben Feststoff, der sich leicht wie folgt kristallisieren lässt:
42 mg Komplex **K3** werden in 1-2 ml Toluol gelöst. In die gelbe klare Lösung werden 6 ml Heptan zugegeben. Die Lösung wird nicht trüb. Man reduziert das Volumen um ein Drittel und es fällt ein gelber Feststoff aus. Man gibt erneut etwas Toluol dazu und erwärmt bei 60 °C auf dem Wasserbad. Die fast klare Lösung wird über Celite filtriert und das klare gelbe Filtrat wird bei 3 °C in den Kühlschrank gestellt. Nach 4 Tagen haben sich gelbbraune, röntgenfähige Kristalle gebildet.
¹H NMR (400 MHz, C₆D₆) δ 8,07 (m, 1H, CH arom), 7,98 (m, 1H, CH arom), 7,64-7,53 (m, 2H, CH arom), 7,23-7,12 (m, 3H, CH arom), 6,98 (m, 3H, CH arom), 4,49-4,36 (m, 2H, Cp oder vinyl), 4,36-4,28 (m, 1H, Cp oder vinyl), 4,10 (m, 0,5H, Cp oder vinyl), 4,08-4,00 (m, 2H, Cp oder vinyl), 3,88-3,81 (m, 2H, Cp oder vinyl), 3,75 (m, 1,5H, Cp oder vinyl), 3,59 (m, 1H, Cp oder vinyl), 3,13 (s, 1H, CH₃), 3,05 (s, 1,5H, CH₃), 2,99 (s, 0,5H, CH₃), 1,33-1,16 (m, 18H, tBu). ³¹P NMR (161 MHz, C₆D₆) δ = 48,83 (d, Hz, J = 17,4 Hz), 47,8 (d, Hz, J = 17,4 Hz), 47,23 (s), 46,54 (s).
HRMS (ESI) m/z⁺berechnet für C₃₅H₄₁FeNO₂P₂Pd (M+Na)⁺ 754,09044.
Es handelt sich um eine Isomerenmischung, die im ³¹P NMR 6 verschiedene Phosphorsignale zeigt und auf 3 diastereomere Komplexe hinweist.
Die Kristallstruktur besteht aus zwei diastereomeren Komplexen.

### Hochdruckexperimente

Einsatzstoffe:
Methanol (MeOH)

Di-n-Buten ist ein Isomerengemisch von C8-Olefinen, das aus der Dimerisierung von Gemischen aus 1-Buten, cis-2-Buten und trans-2-Buten entsteht. Technisch werden der Regel Raffinat II oder Raffinat III-Ströme einer katalytischen Oligomerisierung unterworfen, wobei die enthaltenen Butane (n/iso) unverändert hervorgehen und die enthaltenen Olefine ganz oder Teilweise umgesetzt werden. Neben dem dimeren Di-n-Buten, entstehen in der Regel auch höhere Oligomere (Tributen C12, Tetrabuten C16), die nach der Reaktion destillativ abgetrennt werden.

Ein industriell praktiziertes Verfahren zur Oligomerisierung von C4-Olefinen ist der sogenannte "OCTOL-Prozess". Innerhalb der Patentliteratur beschreibt beispielsweise DE102008007081A1 eine auf dem OCTOL-Prozess beruhende Oligomerisierung. EP1029839A1 befasst sich mit der Fraktionierung der in dem OCTOL-Prozess entstehenden C8-Olefine.
Technisches Di-n-Buten besteht in der Regel 5 bis 30 % aus n-Octenen, 45 bis 75 % aus 3-Methylheptenen und zu 10 bis 35 % aus 3,4-Dimethylhexenen. Bevorzuge Ströme enthalten 10 bis 20 % n-Octene, 55 bis 65 % aus 3-Methylheptene und 15 bis 25 % 3,4-Dimethylhexenen.

PTSA bezeichnet im Folgenden immer das para-Toluolsulfonsäure-Monohydrat.

### Allgemeine Versuchsbeschreibung für Reaktionen im Batchversuch:

Die entsprechenden Mengen an Substrat, Palladiumsalz, Säure und Alkohol werden unter Argon und Magnetrührung in einem 50 ml-Schlenkgefäß vermischt.

Ein 100 ml Stahlautoklav der Firma Parr versehen mit einem Gaseinlass und einem Gasauslassventil, einem digitalem Druckaufnehmer, einem Temperaturfühler und einem Kugelhahn sowie einer eingebauten Kapillare zur Probennahme wird dreimal mittels Vakuum und Argonspülung von Sauerstoff befreit. Anschließend wird die Reaktionslösung aus dem Schlenkgefäß mittels einer Kapillare in den Autoklaven im Argongegenstrom durch den Kugelhahn befüllt. Anschließend wird entweder bei Raumtemperatur die entsprechende Menge an CO aufgepresst und dann auf Reaktionstemperatur hochgeheizt (Reaktionen, die nicht unter konstantem Druck gefahren werden) oder es wird erst auf Reaktionstemperatur hochgeheizt und dann wird das CO über eine Bürette, die mittels eines Druckminderers mit dem Autoklaven verbunden ist, aufgepresst. Diese Bürette wird dann noch auf ca. 100 bar mit CO befüllt und liefert während der Reaktion das benötigte CO bei einem konstanten Druck nach. Diese Bürette hat ein Totvolumen von ca. 30 ml und ist mit einem digitalen Druckaufnehmer versehen. Dann wird die Reaktion bei der benötigten Temperatur die entsprechende Zeit unter Rührung durchgeführt. Hierbei werden mittels einer Software (Specview der Firma SpecView Corporation) und einem Prozesscontroller der Firma Parr 4870 sowie einem 4875 Powercontroler Daten über den Druckverlauf im Autoklaven und in der Gasbürette aufgezeichnet. Aus diesen werden Exceltabellen generiert, aus denen später Diagramme erstellt werden, die Gasverbräuche und damit Umsätze über die Zeit darstellen. Falls benötigt, werden über die Kapillare über die GC Proben gesammelt und analysiert. Hierzu wird vor der Reaktion eine geeignete exakte Menge (2-10 ml) Isooctan als interner Standard mit in das Schlenkgefäß gegeben. Diese geben auch Auskunft über den Reaktionsverlauf. Am Ende der Reaktion wird der Autoklav auf Raumtemperatur heruntergekühlt, der Druck vorsichtig abgelassen, falls nötig Isooctan als interner Standard hinzugefügt und eine GC-Analyse bzw. bei neuen Produkten auch eine GC MS Analyse durchgeführt.

### Allgemeine Versuchsvorschrift für Autoklavenversuche in Glasvials:

Es wird ein 300ml Reaktor der Fa. Parr verwendet. Diesem angepasst ist ein selbstgefertigter Aluminiumblock entsprechender Dimension welcher zu Heizen mittels handelsüblicher Magnetrührer z.B. der Firma Heidolph geeignet ist. Für das Innere des Autoklaven wurde eine runde Metallplatte der Stärke von ca. 1,5 cm angefertigt die 6 Bohrungen enthält, die dem Außendurchmesser der Glasvials entsprechen. Diesen Glasvials angepasst werden sie mit kleinen Magnetrührern bestückt. Diese Glasvials werden mit Schraubkappen und geeigneten Septen versehen und mit einer in der Glasbläserei angefertigten Spezialapparatur unter Argon mit den entsprechenden Reaktanden, Lösungsmittel und Katalysatoren und Additiven befüllt.

Hierzu werden 6 Gefäße gleichzeitig befüllt, dies ermöglicht die Durchführung von 6 Reaktionen bei gleicher Temperatur und gleichem Druck in einem Experiment. Dann werden diese Glasgefäße mit Schraubkappen und Septen geschlossen und es wird jeweils ein kleine Spritzenkanüle geeigneter Größe durch die Septen gestochen. Dies ermöglicht später in der Reaktion den Gasaustausch. Diese Vials werden nun in der Metallplatte platziert und diese wird unter Argon in den Autoklaven überführt. Der Autoklav wird mit CO gespült und bei Raumtemperatur mit dem vorgesehenen CO-Druck befüllt. Dann wird mittels dem Magnetrührer unter Magnetrührung auf Reaktionstemperatur erhitzt und die Reaktion die entsprechende Zeit durchgeführt. Anschließend wird auf Raumtemperatur heruntergekühlt und der Druck langsam abgelassen. Anschließend wird der Autoklav mit Stickstoff gespült. Die Vials werden dem Autoklaven entnommen und mit einer definierten Menge eines geeigneten Standards versetzt. Es erfolgt eine GC Analyse mit dessen Ergebnisse Ausbeuten und Selektivitäten bestimmt wurden.

Der Versuch kann auf entsprechende Weise auch in einem 600 ml Reaktor der Fa. Parr durchgeführt werden, wobei eine Metallplatte mit 12 Bohrungen eingesetzt wird, die mit 12 Reaktionsgefäßen beladen wird.

### Analytik:

GC Analytik 2-Octen: Für die GC Analyse wird ein Chromatograph der Firma Agilent GC Agilent 7890A mit einer 30 m HP5-Säule verwendet. Temperaturprofil: 35 °C, 10 min; 10 °C/min zu 200 °C; das Injektionsvolumen beträgt 1 µl mit einem Split von 50:1. Retentionszeiten für 2-Octen und Produkte: 10.784-13.502 min
Die aus 2-Octen gebildeten Ester werden im Folgenden als MINO bezeichnet (Methylisononanoat).
Retentionszeit für Etherprodukte unbekannter Isomerenverteilung: 15.312, 17.042, 17.244, 17.417 min
Retentionszeit für iso-C9-Ester 19.502-20.439 min (Hauptpeak: 19.990 min)
Retentionszeit für n-C9-Ester: 20.669, 20.730, 20.884, 21.266 min.

Das n/iso-Verhältnis gibt das Verhältnis von endständig zu Estern umgesetzten Olefinen zu innenständig zu Estern umgesetzten Olefinen an.

Die im Folgenden angegebenen n-Selektivitäten beziehen sich auf den Anteil der endständigen Methoxycarbonylierung bezogen auf die Gesamtausbeute an Methoxycarbonylierungsprodukten.

Soweit nicht anders angegeben, beziehen sich im Folgenden relative Stoffmengen in mol-% auf die Stoffmenge an Olefin (Substrat).

### Methoxycarbonylierung von 2-Octen mit unterschiedlichen Liganden

Ein 25 mL Schlenkgefäß wurde mit einer Stammlösung aus Pd(acac)₂ (1.95 mg, 6.4 µmol), MeSO₃H (6.4 µL, 98.55 µmol) und MeOH (10 mL, 24.69 mmol) befüllt. Ein 4 mL-Fläschchen wurde mit 0.16 mol-% des angegebenen Liganden (bezogen auf die Stoffmenge an 2-Octen) befüllt und ein Magnetrührstab hinzugegeben. Danach wurden 1.25 mL der klaren gelben Stammlösung und 2-Octen (315 µL, 2mmol) mit einer Spritze injiziert. Die molaren Anteile bezogen auf die Stoffmenge von 2-Octen betragen dann für Pd(acac)₂ 0.04 mol% und für MeSO₃H 0.6 mol%. Das Fläschchen wurde auf einen Probenhalter gesetzt, der wiederum unter Argon-Atmosphäre in einen 300 mL Autoklaven der Fa. Parr Instruments eingesetzt wurde. Nach dreimaligem Spülen des Autoklaven mit Stickstoff wurde der CO-Druck auf 40 bar eingestellt. Die Reaktion lief bei 120 °C 5 Stunden. Nach Abschluss der Reaktion wurde der Autoklav auf Raumtemperatur abgekühlt und vorsichtig entspannt. Isooctan wurde als interner GC-Standard zugesetzt. Ausbeute an Methylnonanoat und Regioselektivität wurden mittels GC bestimmt. Die Ergebnisse sind in nachfolgender Tabelle zusammengefasst:

| Beispiel | Ligand | Ausbeute | n/iso-Verhältnis |
|---|---|---|---|
| 1* | 1,1'-bis-(tert-butylphenylphosphino)-ferrocen | 85% | 83/17 |
| 2 (VB) | 1-(diphenylphosphino)-1'-(diadamantylphosphino)-ferrocen | 53% | 75/25 |
| 3 (VB) | 1-(diphenylphosphino)-1'-(dicyclohexylphosphino)-ferrocen | 36% | 75/25 |
| 4 (VB) | 1,1'-bis-(iso-propylphenylphosphino)-ferrocen | 55% | 77/23 |
| 5 (VB) | 1-(diphenylphosphino)-1'-(di-isopropylphosphino)-ferrocen | 47% | 73/27 |
| 6 (VB) | 1-(diphenylphosphino)-1'-(tert-butylphenylphosphino)-ferrocen | 65% | 70/30 |
| 7 (VB) | 1,1'-bis(di-(1,4-di-trifluormethyl-phenyl)-phosphino)-ferrocen | 0% | - |

| | | | |
|---|---|---|---|
| VB: Vergleichsbeispiel *: erfindungsgemäßes Beispiel | | | |

Dieser Versuch zeigt, dass mit dem erfindungsgemäßen Liganden 1,1'-bis-(tert-butylphenylphosphino)-ferrocen eine deutlich höhere Ausbeute und eine größere n/iso-Spezifität erzielt werden kann als mit strukturell ähnlichen, aus dem Stand der Technik bekannten Ferrocenylliganden.

### Methoxycarbonylierung von Di-n-Buten mit unterschiedlichen Liganden

Ein 50 mL Schlenkgefäß wurde mit einer Stammlösung aus [Pd(acac)₂] (3.9 mg, 12.9 µmol), MeSO₃H (Methansulfonsäure) (13 uL, 197.1 µmol) und MeOH (20 mL) befüllt. Ein 4 mL-Fläschchen wurde mit 0.16 mol% des angegebenen Liganden (bezogen auf die Stoffmenge an Di-n-Buten) befüllt und ein Magnetrührstab hinzugegeben. Danach wurden 1.25 mL der klaren gelben Stammlösung und Di-n-Buten (315 µL, 2mmol) mit einer Spritze injiziert. Die molaren Anteile bezogen auf die Stoffmenge von 2-Octen betragen dann für Pd(acac)₂ 0.04 mol% und für MeSO₃H 0.6 mol%. Das Fläschchen wurde auf einen Probenhalter gesetzt, der wiederum unter Argon-Atmosphäre in einen 600 mL Parr-Autoklaven eingesetzt wurde. Nach dreimaligem Spülen des Autoklaven mit Stickstoff wurde der CO-Druck auf 40 bar eingestellt. Die Reaktion lief bei 120 °C 20 Stunden. Nach Abschluss der Reaktion wurde der Autoklav auf Raumtemperatur abgekühlt und vorsichtig entspannt. Isooctan wurde als interner GC-Standard zugesetzt. Ausbeute an Methylisononanoat (MINO) und Regioselektivität wurden mittels GC bestimmt. Die Ergebnisse sind in nachfolgender Tabelle zusammengefasst:

| Beispiel | Ligand | Ausbeute | n/iso-Verhältnis |
|---|---|---|---|
| 1* | 1,1'-bis-(tert-butylphenylphosphino)-ferrocen | 73% | 86/14 |
| 2 (VB) | 1,2-bis-(di-tert-butyl-phosphino-methyl)-benzol | 49% | 94/6 |
| | | | |
| 3 (VB) | 1-(di-tert-butylphosphino)-1'-(diphenylphosphino)-ferrocen | 18% | 79/21 |
| 4 (VB) | 1-(diphenylphosphino)-1'-(di-isopropylphosphino)-ferrocen | 12% | 78/22 |
| 5 (VB) | 1-(diphenylphosphino)-1'-(tert-butylphenylphosphino)-ferrocen | 16% | 77/23 |
| 6 (VB) | 1,1'-bis-(iso-propylphenylphosphino)-ferrocen | 22% | 80/20 |

| | | | |
|---|---|---|---|
| VB: Vergleichsbeispiel *: erfindungsgemäßes Beispiel | | | |

Auch bei diesem Versuch werden mit dem erfindungsgemäßen Liganden eine deutlich bessere Ausbeute bei höherer n/iso-Selektivität erzielt als mit strukturell ähnlichen Ferrocenylliganden oder dem bekannten Liganden DTBPMB.

### Methoxycarbonylierung von Di-n-Buten mit präformierten Komplexen

Ein 100 ml Stahlautoklav wird unter Argon mit 0.04 mol-% des angegebenen Komplexes und 1,1'-bis-(tert-butylphenylphosphino)-ferrocen (29.6 mg, 0.12 mol%) befüllt. Anschließend werden MeOH (30 ml) und Di-n-buten (7,54 ml, 48 mmol) und PTSA (54,7 mg, 0.6 mol%) hinzugefügt. Der Autoklav wird bei Raumtemperatur mit 40 bar CO der Reinheit 4.7 befüllt und die Reaktion wird 20 Stunden bei 120 °C durchgeführt. Anschließend wird der Autoklav heruntergekühlt und der Druck langsam abgelassen. Der Autoklaveninhalt wird in ein Schlenkgefäß überführt. Es werden 5 ml Isooctan als interner Standard zugesetzt und die Ausbeute an Methylisononanoat (MINO) und Regioselektivität mittels GC Analyse bestimmt. Die Ergebnisse sind in nachfolgender Tabelle zusammengefasst:

| Komplex | Struktur | Ausbeute | n/iso-Selektivität |
|---|---|---|---|
| K1* | 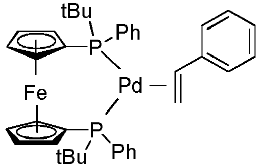 | 76% | 87/13 |
| K2* | 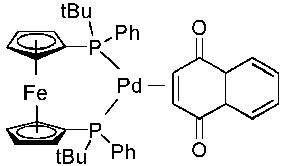 | 72% | 87/13 |
| K3* | 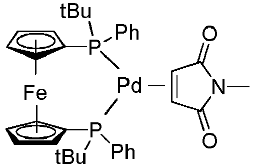 | 72% | 87/13 |

| | | | |
|---|---|---|---|
| *: erfindungsgemäße Beispiele | | | |

Dieser Versuch zeigt, dass mit den präformierten Komplexen ebenso gute Ergebnisse erzielt werden können wie mit den *in situ* gebildeten Komplexen.

## Patentansprüche

1. Verfahren umfassend die Verfahrensschritte:
a) Vorlegen einer ethylenisch ungesättigten Verbindung;
b) Zugabe einer Verbindung gemäß Formel (**1**) 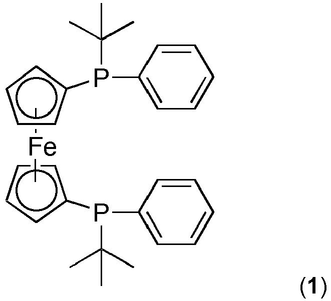 und einer Verbindung, welche Pd umfasst,
oder Zugabe eines Komplexes umfassend Pd und eine Verbindung gemäß Formel (**1**) 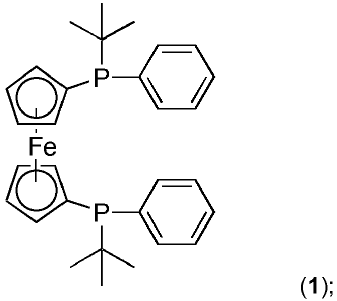
c) Zugabe eines Alkohols;
d) Zuführen von CO;
e) Erwärmen des Reaktionsgemisches, wobei die ethylenisch ungesättigte Verbindung zu einem Ester umgesetzt wird.

2. Verfahren nach Anspruch 1,
wobei die ethylenisch ungesättigte Verbindung ausgewählt ist aus Ethen, Propen, 1-Buten, *cis-* und/oder *trans*-2-Buten, iso-Buten, 1,3-Butadien, 1-Penten, *cis-* und/oder *trans*-2-Penten, 2-Methyl-1-buten, 3-Methyl-1-buten, 2-Methyl-2-buten, Hexen, Tetramethylethylen, Hepten, 1-Octen, 2-Octen, Di-n-Buten, oder Mischungen davon.

3. Verfahren nach einem der Ansprüche 1 bis 2,
wobei die ethylenisch ungesättigte Verbindung 8 bis 22 Kohlenstoffatome umfasst.

4. Verfahren nach einem der Ansprüche 1 bis 3,
wobei die Verbindung, welche Pd umfasst, in Verfahrenschritt b) ausgewählt ist aus Palladiumdichlorid, Palladium(II)-acetylacetonat, Palladium(II)-acetat, Dichloro(1,5-cyclooctadiene)palladium(II), Bis(dibenzylideneaceton)palladium, Bis(acetonitrile)dichloropalladium(II), Palladium(cinnamyl)dichlorid.

5. Verfahren nach einem der Ansprüche 1 bis 4,
wobei der Alkohol in Verfahrensschritt c) ausgewählt ist aus Methanol, Ethanol, 1-Propanol, 1-Butanol, 1-Pentanol, 1-Hexanol, 2-Propanol, *tert*-Butanol, 3-Pentanol, Cyclohexanol, Phenol, oder Mischungen davon.

## Claims

1. Process comprising the following process steps:
a) initially charging an ethylenically unsaturated compound;
b) adding a compound of formula 1 and a compound comprising Pd,
or adding a complex 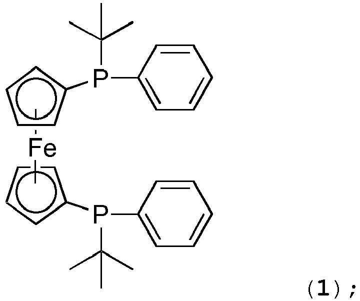 comprising Pd and a compound of formula (1) 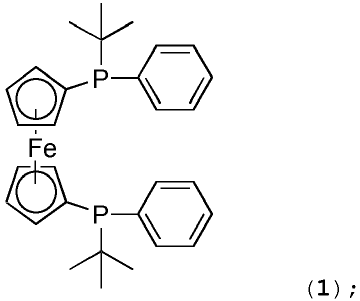
c) adding an alcohol;
d) feeding in CO;
e) heating the reaction mixture, with conversion of the ethylenically unsaturated compound to an ester.

2. Process according to Claim 1,
wherein the ethylenically unsaturated compound is selected from ethene, propene, 1-butene, *cis-* and/or *trans*-2-butene, isobutene, 1,3-butadiene, 1-pentene, *cis-* and/or *trans*-2-pentene, 2-methyl-1-butene, 3-methyl-1-butene, 2-methyl-2-butene, hexene, tetramethylethylene, heptene, 1-octene, 2-octene, di-n-butene, or mixtures thereof.

3. Process according to either of Claims 1 and 2,
wherein the ethylenically unsaturated compound comprises 8 to 22 carbon atoms.

4. Process according to any of Claims 1 to 3,
wherein the compound comprising Pd in process step b) is selected from palladium dichloride, palladium(II) acetylacetonate, palladium(II) acetate, dichloro(1,5-cyclooctadiene)palladium(II), bis(dibenzylideneacetone)palladium, bis(acetonitrile)dichloropalladium(II), palladium(cinnamyl) dichloride.

5. Process according to any of Claims 1 to 4,
wherein the alcohol in process step c) is selected from methanol, ethanol, 1-propanol, 1-butanol, 1-pentanol, 1-hexanol, 2-propanol, *tert*-butanol, 3-pentanol, cyclohexanol, phenol, or mixtures thereof.

## Revendications

1. Procédé comprenant les étapes de procédé suivantes :
a) le chargement d'un composé éthyléniquement insaturé ;
b) l'ajout d'un composé selon la formule (1) 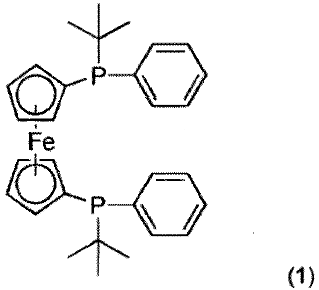 et d'un composé qui comprend Pd,
ou l'ajout d'un complexe comprenant Pd et un composé selon la formule (1) 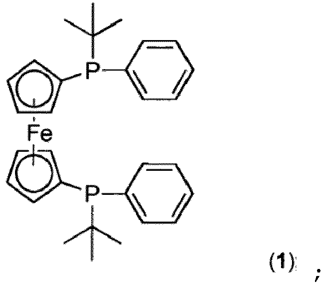
c) l'ajout d'un alcool ;
d) l'introduction de CO ;
e) le chauffage du mélange réactionnel, le composé éthyléniquement insaturé étant transformé en un ester.

2. Procédé selon la revendication 1, dans lequel le composé éthyléniquement insaturé est choisi parmi l'éthène, le propène, le 1-butène, le *cis-* et/ou le *trans*-2-butène, l'iso-butène, le 1,3-butadiène, le 1-pentène, le *cis-* et/ou le *trans*-2-pentène, le 2-méthyl-1-butène, le 3-méthyl-1-butène, le 2-méthyl-2-butène, l'hexène, le tétraméthyléthylène, l'heptène, le 1-octène, le 2-octène, le di-n-butène ou leurs mélanges.

3. Procédé selon l'une quelconque des revendications 1 à 2, dans lequel le composé éthyléniquement insaturé comprend 8 à 22 atomes de carbone.

4. Procédé selon l'une quelconque des revendications 1 à 3, dans lequel le composé qui comprend Pd à l'étape de procédé b) est choisi parmi le dichlorure de palladium, l'acétylacétonate de palladium (II), l'acétate de palladium (II), le dichloro(1,5-cyclooctadiène)palladium (II), le bis(dibenzylidène-acétone)palladium, le bis(acétonitrile)dichloropalladium (II), le dichlorure de palladium(cinnamyle).

5. Procédé selon l'une quelconque des revendications 1 à 4, dans lequel l'alcool à l'étape de procédé c) est choisi parmi le méthanol, l'éthanol, le 1-propanol, le 1-butanol, le 1-pentanol, le 1-hexanol, le 2-propanol, le *tert*-butanol, le 3-pentanol, le cyclohexanol, le phénol ou leurs mélanges.
